# EUROPEAN PATENT APPLICATION

(11) **EP 4 494 485 A1**
(43) Date of publication of application: **22.01.2025**
(21) Application number: 22931940.5
(22) Date of filing: 14.03.2022
(51) Int. Cl.: A23L 33/13, A61K 31/706, A61P 1/02, A61Q 11/00

(54) **AGENT FOR ORAL DISEASES**

(71) Applicant: Mirailab Bioscience Inc., Tokyo 104-0061 (JP)
(72) Inventor: TANAKA, Megumi, Chigasaki-shi, Kanagawa 253-0054 (JP); TANAKA, Tsunemaru, Chigasaki-shi, Kanagawa 253-0054 (JP)
(74) Representative: IPAZ
(86) International application number: PCT/JP2022/011206
(87) International publication number: WO 2023/175655

(57) **Abstract**

[PROBLEM] To provide a medicine for oral disease that is safe even when a subject takes the medicine over a long period of time and can effectively improve oral diseases.

[SOLUTION] The present invention is a medicine for oral disease containing nicotinamide mononucleotide as an active ingredient.

## Description

### Technical Field

The present invention relates to a medicine for oral disease containing nicotinamide mononucleotide as an active ingredient.

### Background Art

Periodontal diseases are said to affect about 80% of adults in Japan, and are one of the most widely spread diseases in the world. Therefore, although many people are very interested in the periodontal diseases, it cannot be said that their prophylaxis and treatment are still effectively performed now. These diseases are an inflammatory diseases caused by toxin that is a product of a mass (plaque) of periodontal pathogens (Porphyromonas gingivalis, Tannerella forsythia, Fusobacterium nucleatum, Treponema denticola, etc.) that exist in the plaque or biofilm accumulated in periodontal pockets, which are gaps between teeth and gums. Therefore, the main cause of the periodontal diseases is plaque, but the involvement of hormones, stress, smoking, drugs, lack of exercise, lack of sleep, and the like has been pointed out as indirect causes that worsen the periodontal diseases.

Generally, the periodontal diseases are divided into gingivitis and periodontitis (pyorrhea) depending on the progression of the disease. Gingivitis is an inflammation limited to the gingiva in the early stage of the periodontal disease. The gingivitis exhibits symptoms such as red swelling and bleeding due to inflammation at the border between the teeth and the gums, and is common in young people in their twenties and under. On the other hand, the periodontitis is common in people in their thirties and over. As the inflammation of the gingivitis continues, not only the gums are swollen and bled, but also the periodontal ligament fibers supporting the teeth are destroyed. Then, the alveolar bones decay, and the periodontal pocket progresses deeper toward the inside. As a result, the gums become thinner and the teeth gradually become loose, and finally the teeth fall out. Severe periodontitis is the main cause of losing teeth.

Recent researches have gradually revealed that when the periodontal diseases make it difficult to chew food, this is the cause of cardiovascular diseases, diabetes, digestive system diseases, and the like. Furthermore, it is said that when a patient suffers from the periodontal diseases, periodontal pathogens are carried in blood and travel throughout the body via the blood vessels exposed in the gums, and the patient is secondarily likely to be more susceptible to lifestyle-related diseases, respiratory diseases, birth of a low-weight infant, premature birth, and the like. As mentioned above, the periodontal diseases locally affect the teeth and periodontal tissues, and may also possibly cause systemic lesions and symptoms.

Furthermore, as the periodontal disease progresses, periodontal pathogens accumulate in the periodontal pockets, and produce pus, resulting in halitosis. This halitosis caused by the periodontal diseases emits a rotten onion-like smell of methyl mercaptan, and has characteristics of a much stronger smell than the general physiological halitosis caused by dirty tongue (coated tongue). A large part of the physiological halitosis can be eliminated, for example, by cleaning the tongue and removing the coated tongue. However, in order to fundamentally eliminate the halitosis caused by the periodontal disease, it is necessary to treat the periodontal disease, which is the root cause of the halitosis.

As mentioned above, because the periodontal diseases are related to the health of the whole body, it is important to receive an appropriate treatment early if a patient is affected. Currently, the basis of a method for treating the periodontal diseases is plaque control. As the plaque control, physical methods such as brushing, flossing, scaling, and root planing and chemical methods using a drug are typical.

In the basic treatment of relatively mild periodontal diseases, brushing, scaling, and root planing are the mainstream. On the other hand, for moderately or highly advanced periodontal diseases or areas where scaling and root planing are difficult to perform, a combination therapy of scaling, root planing and a local administration of a drug into the periodontal pockets is considered effective. As the drug, drugs containing components having an antimicrobial action and an anti-inflammatory action in a composite manner, such as a tetracycline presteron dental ointment, and a hinoporon oral ointment (manufactured by Showa Yakuhin Kako Co., Ltd., product name) that contains hinokitiol having an antimicrobial action and hydrocortisone acetate having an anti-inflammatory action, have been widely used, in addition to antibacterial drugs such minocycline hydrochloride dental ointment. However, long-term use of antimicrobial agents, especially antibiotics, may possibly lead to, for example, the emergence of resistant bacteria and the occurrence of microbial substitution. Therefore, it is desirable to avoid long-term use as much as possible.

Therefore, various drugs for periodontal diseases that can be used for a long period of time have been reported. For example, a toothpaste composition for periodontal diseases has been reported, wherein the toothpaste composition for periodontal diseases includes: (A) 0.1 to 15% by mass of hydrogel particles having an average particle size of 100 to 500 µm, where the hydrogel particles contain, as a medicinal component, polyphenols as a water-soluble medicinal component, which are dispersed or emulsified in a content of 0.001 to 10% by mass in the component (A) and contain a polymer selected from polyvinylpyrrolidone, hydroxyethyl cellulose, and hydroxypropylcellulose, which forms a water-insoluble complex with the water-soluble medicinal component; (B) a binding agent; and (C) 5 to 40% by mass of water; and agar as a gel-forming agent, the content of which is 0.5 to 5% by mass in the component (A) (Patent Literature 1).

As an agent containing a novel lactic acid bacterium strain, which is safe in the oral cavity, a prophylactic and/or therapeutic agent for diseases in the oral cavity has been reported, wherein the prophylactic and/or therapeutic agent contains, as an active ingredient, one kind or two kinds or more of lactic acid bacteria selected from *Lactobacillus crispatus* YIT 12319 (FERM BP-11500), *Lactobacillus fermentum* YIT 12320 (FERM BP-11501), *Lactobacillus gasseri* YIT 12321 (FERM BP-11502), and *Streptococcus mitis YIT 12322* (FERM BP-11503) (Patent Literature 2).

In addition to the above, as an agent containing amino acid constituting protein in the body, a composition for oral cavity for preventing periodontal diseases has been reported, wherein the composition contains one kind or two kinds or more of amino acids selected from glycine, alanine, leucine, histidine, and proline, and contains, as an active ingredient that neutralizes bacterial endotoxin of periodontal diseases, a component in which the number of constituent amino acids is 1 to 4 (Patent Literature 3).

### Citation List

### Patent Literature

Patent Literature 1: JP 2014-94959 A
Patent Literature 2: JP 5982376 B
Patent Literature 3: JP 10-158131 A

### Summary of Invention

### Technical Problem

An object of the present invention is to provide a medicine for oral disease that is safe for use over a long period of time and useful for preventing and treating periodontal diseases and the like.

### Solution to Problem

As a result of serious study for solving the above-described problems, the inventor discovered that nicotinamide mononucleotide, which is an intermediate metabolite related to biosynthesis of coenzyme nicotinamide adenine dinucleotide (NAD), has a beneficial prevention and improving effect on periodontal disease and the like, and completed the present invention.

The present invention is as follows.
[1] A medicine for oral disease, comprising
   nicotinamide mononucleotide as an active ingredient.
[2] The medicine for oral disease according to [1],
   wherein the oral disease is periodontal disease, halitosis, or xerostomia.
[3] The medicine for oral disease according to [1]or [2],
   wherein the medicine for oral disease is a food product for improving oral disease.
[4] The medicine for oral disease according to [1]or [2],
   wherein the medicine for oral disease is a medicinal product for improving oral disease.
[5] The medicine for oral disease according to any one of [1] to [4],
   wherein a daily amount per adult of the nicotinamide mononucleotide to be applied is 1 mg to 500 mg.
[6] A method for improving oral disease (excluding medical practice to human), the method comprising
   applying an effective dose of nicotinamide mononucleotide to a target in need thereof. Advantageous Effects of Invention

The medicine for oral disease according to the present invention is effective for preventing or treating oral diseases such as periodontal disease. In addition, since nicotinamide mononucleotide, which is an intermediate metabolite related to in vivo NAD⁺ biosynthesis, is contained as an active ingredient, the medicine for oral disease according to the present invention is safe and can be used over a long period of time.

### Brief Description of Drawings

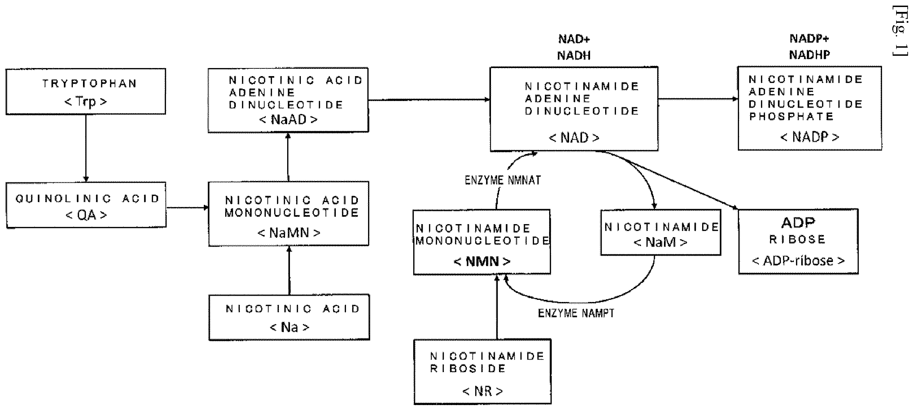

Fig. 1 is an explanatory diagram illustrating metabolic pathways related to niacin (general term for nicotinamide and nicotinic acid).

### Description of Embodiments

The medicine for oral disease according to the present invention contains nicotinamide mononucleotide (including a salt thereof) as an active ingredient and provides an improving effect on oral diseases such as periodontal disease. In the present invention, the improvement of oral diseases includes not only the improvement of oral diseases in a narrow sense, but also the alleviation of various symptoms developed from oral diseases, the prevention of oral diseases, and stopping and delaying progress. The detailed reason for obtaining such an action effect by containing nicotinamide mononucleotide as an active ingredient is currently under consideration. However, it is considered that the nicotinamide mononucleotide accelerates "sirtuins" represented by NAD⁺-dependent deacetylases Sirt1 and Sirt3 and consequently leads to phenomena such as an increase in resistance of a periodontal tissue and an increase in a secretion amount of saliva, thereby providing an effect on oral diseases such as periodontal disease. Thus, the medicine for oral disease according to the present invention that provides an improving effect on oral diseases can be used as a medicinal product (including a quasi-drug) and a food product such as a functional food. The following describes the present invention in detail.

The nicotinamide mononucleotide (chemical formula: C₁₁H₁₅N₂O₈P) is a compound produced in bodies of many organisms including human, and expressed with a structural formula [Chem. 1] below. The nicotinamide mononucleotide is generally referred to as NMN, and known as an intermediate metabolite involved in a biosynthesis of coenzyme NAD⁺.

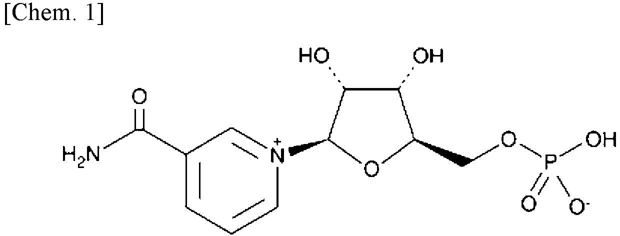

The nicotinamide mononucleotide, which is contained as an active ingredient of the medicine for oral disease, is produced in an NAD metabolic pathway by liver tissues, that is, a pathway involved in a synthesis of a nicotinamide adenine dinucleotide (NAD) from a quinolinic acid through a kynurenine pathway, in vivo. This will be specifically described with reference to Fig. 1. Fig. 1 is an explanatory drawing illustrating a metabolic pathway involved in niacin (generic term of a nicotinamide and a nicotinic acid) known as vitamin B₃. The nicotinic acid ingested through a meal is absorbed by the liver to be converted into nicotinamide, and the nicotinamide is supplied to the whole body via a blood flow. The cells each absorb the nicotinamide from the blood, and convert it into the NAD and an NADP to use them. The nicotinamide is biosynthesized also from a tryptophan.

As illustrated in Fig. 1, in vivo, when the tryptophan is a starting material, the tryptophan is converted into the quinolinic acid (QA) through the kynurenine pathway as a tryptophan metabolic pathway, and further converted into a nicotinic acid mononucleotide (NaMN). Meanwhile, when the nicotinic acid (Na) is the starting material, the nicotinic acid is directly converted into the NaMN. Afterwards, the NaMN is interconverted into the NAD, a nicotinamide (NaM), and the nicotinamide mononucleotide in a NAD cycle through a nicotinic acid adenine dinucleotide (NaAD). The nicotinamide (NaM) is converted into the nicotinamide mononucleotide by a nicotinamide phosphoribosyltransferase (NAMPT), subsequently, the nicotinamide mononucleotide is converted by a nicotinamide mononucleotide adenyltransferase (NMNAT) to generate the NAD. Note that, the nicotinamide mononucleotide is produced also from a nicotinamide riboside (NR) as an NAD intermediate metabolite.

The nicotinamide mononucleotide includes two types of an α-form and a β-form as optical isomers, and the β-form is used in the present invention. The nicotinamide mononucleotide is obtained by, for example, synthesizing a nicotinamide riboside from the nicotinamide and a ribose (see Bioorg. Med. Chem. Lett., 12, 1135-1137 (2002)), and subsequently, phosphorylating a 5-hydroxyl group of the ribose part (see Chem. Comm., 1999, 729-730). Specifically, for example, first, a reaction solution is prepared by dissolving the nicotinamide and an L-ribose tetraacetate in anhydrous acetonitrile, adding a trimethylsilyl trifluorosulfonic acid by an excessive amount under a nitrogen stream and then stirring at room temperature, and adding methanol to stop the reaction. The above-described reaction solution is poured into a column filled with activated carbon, cleaned with a distilled water, and then eluted with methanol and its product is collected. Next, for a phosphorylation reaction of the 5-hydroxyl group of the L-ribose part of this product, a reaction solution is prepared by dissolving the above-described product in a trimethoxy phosphoric acid, dropping a phosphorus oxychloride below freezing and stirring under the nitrogen stream, adding a sodium hydroxide aqueous solution to neutralize, thus stopping the reaction. A cold acetonitrile-ether solution is added to the above-described reaction solution. Afterwards, a lower layer (water phase) is passed through an anion-exchange resin to collect a reactant, and further purifies the reactant with a cation-exchange resin, thus the high-purity nicotinamide mononucleotide can be collected. The nicotinamide mononucleotide is commercially available from Oriental Yeast Co., ltd. and Bontac Bio-engineering (Shenzhen) Co., Ltd., and those commercial products can be purchased for use.

The medicine for oral disease according to the present invention can be easily manufactured by using nicotinamide mononucleotide alone or by mixing the nicotinamide mononucleotide with other components. The other components are not specifically limited as long as the medicine for oral disease provides the effect of the present invention.

Examples of the other components include medicinal components having an antibacterial and/or an anti-inflammatory action such as chamomile tincture, krameria triandra tincture, myrrh tincture, hinokitiol, chamazulene, azulene, rhatannin , tannin, tocopherol acetate, cetylpyridinium chloride hydrate, glycyrrhizic acid, dipotassium glycyrrhizate, monoammonium glycyrrhizate, tranexamic acid, isopropylmethylphenol, epsilon aminocaproic acid, methyl salicylate, chlorhexidine hydrochloride, benzethonium chloride, lysozyme chloride, sodium chloride, chlorhexidine gluconate, triclosan, allantoin, phellodendron bark extract, sodium lauroyl sarcosinate, hinokitiol, thymol, propolis, clove oil, and canavanine. In addition, for example, various vitamins, trace amounts of elements, citric acid, malic acid, flavoring agents, inorganic salt, and the like, which are common supplemental components in the food product field may be included as other components.

In the present invention, as especially effective other components for enhancing an action of improving the oral diseases, resveratrol is exemplified. Resveratrol is known as an antioxidant contained in, for example, a grape pericarp, red wine, a peanut skin, Polygonum cuspidatum, and Gnetum gnemon. The resveratrol includes resveratrol derivatives such as trans and cis isomers, trans-cis isomer mixtures, dimers, and methylated resveratrol. Generally, the trans isomer that is stable to heat is used for a healthy food product and the like. In addition, resveratrol may be prepared by extraction and purification from any source, or it may be synthetically prepared.

A compounding ratio between resveratrol and nicotinamide mononucleotide is not limited. However, the compounding ration therebetween is preferably adjusted so that an amount of nicotinamide mononucleotide is 1 to 25 parts by mass with respect to 1 to 100 parts by mass of resveratrol in a daily intake for an adult, from the viewpoint of maximizing the effect of the present invention.

The medicine for oral disease according to the present invention can be used for the purpose of improving various oral diseases, but is mainly used for the purpose of improving periodontal diseases, halitosis, or xerostomia (dry mouth).

As mentioned above, the periodontal diseases include gingivitis and periodontitis, both of which are applicable to the present invention. For example, when the medicine for oral disease according to the present invention is applied/embrocated to an area where the periodontal disease is affected, it is expected that various symptoms of periodontal diseases, such as redness, swelling, bleeding, pus from the gums, itchy gums, sticky mouth, and the like, can be alleviated.

As mentioned above, examples of the halitosis mainly include halitosis derived from the periodontal disease and physiological halitosis derived from coated tongue. However, the medicine for oral disease according to the present invention is applicable to both types of the halitosis. In the case of the halitosis derived from the periodontal disease, as mentioned above, as the periodontal disease is improved by the medicine for oral disease according to the present invention, the halitosis is also improved. Moreover, in the case of the physiological halitosis derived from coated tongue, for example, it is expected that as the coated tongue is removed and the medicine for oral disease according to the present invention is orally taken, the symptoms of the halitosis are alleviated.

Xerostomia is a condition in which the oral cavity becomes excessively dry due to a decrease in saliva secretion, resulting in unpleasant symptoms such as sore throat, dry feeling of the oral cavity, sticky saliva, and sticky mouth. When the medicine for oral disease according to the present invention is taken, saliva secretion is promoted, making saliva thinner, and it is expected that symptoms such as the dry feeling of the oral cavity will be alleviated.

A method for manufacturing the medicine for oral disease is not specifically limited, and a common manufacturing method applied for manufacturing the medicine for oral disease according to its form may be appropriately selected. For example, when the form is a dentifrice, the medicine for oral disease can be manufactured by appropriately combining nicotinamide mononucleotide, other medicinal components, and components, such as a polishing agent, a foaming agent, a foaming aid, a surfactant, a polishing agent, an extending agent, a sweetening agent, a preservation agent, a pH adjuster, an adhesive, a colorant a pigment, and a flavoring agent, and uniformly dispersing and mixing them using equipment having sufficient shearing force and mixing force for manufacturing. Note that, nicotinamide mononucleotide as an active ingredient is distributed in the market and is commercially available. Especially, in recent years, a quality management system and a mass production system of nicotinamide mononucleotide have been established, allowing the nicotinamide mononucleotide to be supplied as a food composition material, and further, the stability of nicotinamide mononucleotide as a food composition has been confirmed.

In the pharmaceutical field, the medicine for oral disease according to the present invention can be used as a medicinal product (including a quasi-drug) for improving oral disease and can be applied orally or parenterally. When it is applied orally, a dosage form of the medicine for oral disease is not specifically limited, but can include, for example, a powder, a tablet, a persistent tablet, a chewable tablet, an effervescent tablet, a troche, a buccal tablet, a sublingual tablet, a capsule formulation, a fine granule, a granule, a pill, a dry syrup, a liquid medicine, a suspending agent, a syrup, and an elixir. Among these forms, considering the ease of taking the medicine, the stability of the active ingredient, and the like, the formulation for oral administration such as the powder, the tablet, and the capsule formulation is preferable.

On the other hand, when the medicine for oral disease according to the present invention is applied parenterally, examples of the dosage form of the medicine for oral disease include an external preparation, an injection preparation, and a transfusion. However, the external preparation is preferable because the active ingredient can be applied directly into the oral cavity. Specifically, the external preparation can be a dentifrice (including "paste" in the form of paste, fluid "liquid" with a low viscosity, "liquid" having almost the same viscosity as water, wet powdery "semi-paste", and powdery "powder"), a mouthwash, a mouth spray, an ointment, a cream, or the like.

The medicinal product can appropriately contain a known additive for formulation, which is adequate for the dosage form and pharmacologically allowed, considering physicochemical property, biological property, and similar property. Such an additive for formulation is exemplified by, for example, an excipient (lactose, starch, crystalline cellulose, sodium phosphate, and the like), a solvent (water, soybean oil, saline solution, a nonaqueous solvent for injection, and the like), a binder (starch, gelatin, gum arabic, sodium alginate, carmellose sodium, methylcellulose, ethyl cellulose, hydroxypropyl methylcellulose, hydroxypropyl cellulose, polyvinylpyrrolidone, and the like), a disintegrant (starch, carmellose sodium, and the like), a lubricant (talc, magnesium stearate, calcium stearate, macrogol, sucrose fatty acid ester, and the like), a coating agent (white sugar, HPC, shellac, gelatin, glycerin, hydroxypropyl methylcellulose, hydroxypropyl methylcellulose phthalate, cellulose acetate phthalate, and the like), a stabilizer (sodium bisulfite, sodium thiosulfate, sodium edetate, sodium citrate, ascorbic acid, dibutylhydroxytoluene, and the like), a preservative (methyl parahydroxybenzoate, ethyl parahydroxybenzoate, propyl parahydroxybenzoate, benzyl alcohol, phenol, chlorobutanol, benzalkonium chloride, benzethonium chloride, sodium dehydroacetate, thimerosal, and the like), a viscous agent (methylcellulose, carmellose sodium, chondroitin sulfate, sodium alginate, and the like), a suspending agent (various nonionic surfactant, methylcellulose, carmellose sodium, and the like), an emulsifier (gum arabic, cholesterol, sorbitan sesquioleate, polysorbate 80, sodium lauryl sulfate, and the like), a buffer (citric acid, acetic acid, sodium phosphate, and boric acid), a surfactant (hydrogenated castor oil, polysorbate 80, and the like), a colorant (water-soluble food pigment, lake pigment, and the like), a corrigent (lactose, white sugar, glucose, mannitol, and the like), a scenting agent (aromatic essential oils), a plasticizer (the phthalic acid esters, vegetable oils, polyethylene glycol, and the like).

A dose of the medicinal product cannot be equally specified because it differs depending on, for example, age, sex, and weight of a target that takes the food product, the expected effect, and the symptom. However, as the dose of the medicinal product, the daily amount per adult of the nicotinamide mononucleotide to be administered is ordinarily 1 mg to 500 mg, preferably 5 mg to 250 mg, and more preferably 50 mg to 200 mg. Less than 1 mg possibly fails to provide the effect of the present invention, while more than 500 mg merely provides almost similar effect but causes economic disadvantage. The compounding ratio of the nicotinamide mononucleotide in the medicinal product can be appropriately set in accordance with the dosage form, the number of times of the medicinal product administration, and the like so that the daily amount per adult of the nicotinamide mononucleotide to be administered is within the above range.

The number of times of the administration of the medicinal product can be appropriately set in accordance with, for example, the age, weight, and symptom of the administration target and the dose per administration of the medicinal product. The number of times of the medicinal product administration per day can be exemplified by once to three times.

The medicine for oral disease according to the present invention can be used as a food product. In a case of being used as a food product, the medicine for oral disease can be provided as a food product for improving oral disease in the food product field. By daily ingesting the medicine in the form of a food product, the improving effect on oral diseases can be provided repeatedly and is thus especially effectual for enjoying the effect. The type of the food product as the target of the present invention is not specifically limited, and the target includes a functional food, a food for specified health use, a dietary supplement, a supplement, a food additive, a feed, a care food, a diet therapy food, a therapeutic diet, a diet food, and similar food product in addition to general food products. The form of the food product is not limited, and especially in the case of the functional food, the food for specified health use, and the like, the food product can be processed to be provided in the form of, for example, a powder, a tablet, a pill, a granule, a hard capsule formulation, a soft capsule formulation, a jelly, a liquid medicine, a paste medicine, and a chewing gum.

The intake of the food product is different depending on the type of the food product, age, sex, and weight of a target that takes the food product, the expected effect, and the symptom. However, the daily intake per adult of the nicotinamide mononucleotide contained in the food product is ordinarily 1 mg to 500 mg, preferably 5 mg to 250 mg, and more preferably 50 mg to 200 mg. Less than 1 mg possibly fails to provide the effect of the present invention, while more than 500 mg merely provides almost similar effect but causes economic disadvantage. The compounding ratio of the nicotinamide mononucleotide in the food product can be appropriately set in a range of 100% or less so that the daily amount per adult of the nicotinamide mononucleotide to be administered is within the above range.

The food product is safe, and side effects are not specifically recognized. Accordingly, the food product can be ingested over a long period of time not only for the purpose of treating oral diseases but also for the purpose of preventing oral diseases. Therefore, the food product can be applied not only to targets for whom it is desired to treat oral diseases but also to healthy targets so as not to lead to oral diseases.

Since nicotinamide mononucleotide, as mentioned above, has an oral disease improving effect, the present invention further provides a method for improving oral disease, which is characterized by causing a target in need thereof to take an effective dose of nicotinamide mononucleotide. That is, it is a method for improving the oral disease of the target who takes the medicine by causing the target to take the medicine for oral disease according to the present invention. The target of the ingestion is preferably a mammal such as a human, a mouse, a rat, a rabbit, a dog, a cat, cattle, a horse, a pig, and a monkey, and especially a human is preferable. In the method, the intake of the nicotinamide mononucleotide, the number of intake per day, and similar matters are as described for the medicine for oral disease. The medicine for oral disease can be ingested anytime in any case, and can be ingested by the target over a long period of time. Examples

Hereinafter, the present invention will be specifically described based on Examples. Unless otherwise indicated, the content of each component is indicated by % by mass.

### [Example]

The dentifrice having the formulation shown in Table 1 was prepared by a general method, and was evaluated by the following method.

**[Table 1]**

| ^^1 | Component name | Formulation ratio |
|---|---|---|
| 1 | Water | 62.840% |
| 2 | Glycerin | 20.000% |
| 3 | (Provided) β-NMN | 7.000% |
| 4 | Ethanol | 2.000% |
| 5 | Xylitol | 2.000% |
| 6 | Xanthan gum | 2.000% |
| 7 | Isopentyldiol | 1.000% |
| 8 | Na citrate | 1.000% |
| 9 | Citric acid | 0.020% |
| 10 | Hydroxyapatite | 0.010% |
| 11 | Na polyacrylate | 0.530% |
| 12 | Polyglyceryl-2 laurate | 0.049% |
| 13 | Polyglyceryl-10 laurate | 0.049% |
| 14 | PEG-60 hydrogenated castor oil | 0.600% |
| 15 | Glyceryl caprate | 0.003% |
| 16 | Phenoxyethanol | 0.400% |
| 17 | Flavoring agent | 0.500% |

### Component name Formulation ratio

- 1: Water
- 2: Glycerin
- 3: (Provided) β-NMN
- 4: Ethanol
- 5: Xylitol
- 6: Xanthan gum
- 7: Isopentyldiol
- 8: Na citrate
- 9: Citric acid
- 10: Hydroxyapatite
- 11: Na polyacrylate
- 12: Polyglyceryl-2 laurate
- 13: Polyglyceryl-10 laurate
- 14: PEG-60 hydrogenated castor oil
- 15: Glyceryl caprate
- 16: Phenoxyethanol
- 17: Flavoring agent

### <Evaluation method>

Ten subjects (two subjects in their 30s, two subjects in their 40s, three subjects in their 50s, and three subjects in their 70s) with symptoms of the periodontal disease (gingivitis) were evaluated for their improvement in the periodontal disease. Approximately 1 g of the above dentifrice (containing 7.0% of β-NMN) was placed on a toothbrush, and the subjects brushed their teeth for approximately 10 minutes once every morning and afternoon for seven consecutive days. The dentifrice was kept in a refrigerator except when it was used. As the effect of the above dentifrice in improving the periodontal disease, the effect on bleeding on probing (BOP) and the effect on red complex before and after the above toothbrushing for seven days (29 to 143 days after the start of the toothbrushing) were evaluated as follows.

### 1) Bleeding on probing (BOP)

For each subject, the ratio of bleeding sites on probing was determined for a total of 9 to 30 sites of gingival sulcuses and pockets before and after the above toothbrushing for seven days, and the average of the above ratios for all subjects was calculated to evaluate the above bleeding on probing. The presence or absence of the bleeding on probing was determined by inserting a pocket probe lightly (with a force of about 15 to 20 g) into the gingival sulcuses and pockets, and then pulling it out, followed by checking the presence or absence of bleeding after 20 to 30 seconds.

### 2) Effect on red complex

For each subject, before and after the above toothbrushing for seven days, oral floras were collected as a sample from the gingival sulcus in the oral cavity and the metagenomic analysis was performed at the Oral Microbiome Center. The total number of detected bacteria was set to 1 million, and the ratio of the red complex (Porphyromonas gingivalis, Treponema denticola, Tannerella forsythia), which is considered to be the main cause of the onset and progression of periodontitis among periodontal diseases was determined, and the effect on the red complex was evaluated by calculating the average of the above ratios for all subjects.

### <Results and discussion>

As a result of performing the above evaluation using the above dentifrice, regarding the BOP, the average for all subjects before the toothbrushing was 49%, while the average for all subjects after the toothbrushing for seven days was decreased to 21%.

Furthermore, regarding the red complex, the average before the above toothbrushing was 21%, while the average after the above toothbrushing for seven days was decreased to 11%.

From the above results, it was shown that when the dentifrice of the present invention is used for toothbrushing, the periodontal diseases are improved.

## Claims

1. A medicine for oral disease, comprising
nicotinamide mononucleotide as an active ingredient.

2. The medicine for oral disease according to claim 1,
wherein the oral disease is periodontal disease, halitosis, or xerostomia.

3. The medicine for oral disease according to claim 1 or 2,
wherein the medicine for oral disease is a food product for improving oral disease.

4. The medicine for oral disease according to claim 1 or 2,
wherein the medicine for oral disease is a medicinal product for improving oral disease.

5. The medicine for oral disease according to any one of claims 1 to 4,
wherein a daily amount per adult of the nicotinamide mononucleotide to be applied is 1 mg to 500 mg.

6. A method for improving oral disease (excluding medical practice to human), the method comprising
applying an effective dose of nicotinamide mononucleotide to a target in need thereof.
